# EUROPEAN PATENT SPECIFICATION

(11) **EP 2 235 208 B1**
(45) Date of publication and mention of the grant of the patent: **13.06.2012**
(21) Application number: 08861620.6
(22) Date of filing: 16.12.2008
(51) Int. Cl.: C12Q 1/68

(54) **A METHOD OF DETECTING CRYPTOSPORIDIUM**
VERFAHREN ZUM NACHWEIS VON CRYPTOSPORIDIUM
PROCEDE DE DETECTION DE CRYPTOSPORIDIUM

(30) Priority: 17.12.2007 AU 2007906896
(43) Date of publication of application: 06.10.2010
(73) Proprietor: SYDNEY WATER CORPORATION, incorporated under the Sydney Water Act, 1994 (NSW), Parramatta, New South Wales 2150 (AU)
(72) Inventor: MIKOSZA, Andrew Stanislaw John, Jandakot, Western Australia 6164 (AU)
(74) Representative: Fleck, Barbara
(86) International application number: PCT/AU2008/001845
(87) International publication number: WO 2009/076708

(56) References cited:
- WO-A2-2006/117676
- US-B1- 7 202 027
- KARANIS, P. ET AL.: "Development and preliminary evaluation of a loop-mediated isothermal amplification procedure for sensitive detection of Cryptosporidium oocysts in fecal and water samples" APPLIED AND ENVIRONMENTAL MICROBIOLOGY, vol. 73, no. 17, September 2007 (2007-09), pages 5660-5662, XP007915635
- RYAN UNA ET AL: "Molecular and biological characterization of a Cryptosporidium molnari-like isolate from a guppy (Poecilia reticulata)." APPLIED AND ENVIRONMENTAL MICROBIOLOGY JUN 2004 LNKD- PUBMED:15184187, vol. 70, no. 6, June 2004 (2004-06), pages 3761-3765, XP007915615 ISSN: 0099-2240
- JIANG JIANLIN ET AL: "An evaluation of molecular diagnostic tools for the detection and differentiation of human-pathogenic Cryptosporidium spp" JOURNAL OF EUKARYOTIC MICROBIOLOGY, LAWRENCE, KS, US LNKD- DOI:10.1111/J.1550-7408.2003.TB00623.X, vol. 50, no. Suppl, 1 January 2003 (2003-01-01), pages 542-547, XP009140752 ISSN: 1066-5234 [retrieved on 2005-07-11]
- DATABASE EBI [Online] 4 November 2006 (2006-11-04), "Ricinus communis EST sequencing" XP007915610 retrieved from EMBL Database accession no. EG663005
- DATABASE EBI [Online] 16 June 2004 (2004-06-16), "Apple Functional Genomics grant - NSF 0321702" XP007915611 retrieved from EMBL Database accession no. C0066202
- DATABASE EBI [Online] 24 September 2004 (2004-09-24), "Petunia x hybrida EST collection" XP007915612 retrieved from EMBL Database accession no. CV298710
- DATABASE ebi [Online] 1 November 2007 (2007-11-01), "Saccharomyces cerevisiae oligonucleotide probe" XP007915613 retrieved from EMBL Database accession no. AGD12555
- Panagiotis Karanis: "Cryptosporidiosis: it is more than only an infection" Join International Medicine Meeting 2007. Health Security in the Tropics. 29 November 2007 (2007-11-29), pages 1-44, XP007915614 Retrieved from the Internet: URL:http://www.tm.mahidol.ac.th/jitmm2007/ pdf/301107_Panagiotis.pdf [retrieved on 2010-11-30]
- SMITH H V ET AL: "Tools for investigating the environmental transmission of Cryptosporidium and Giardia infections in humans" TRENDS IN PARASITOLOGY, ELSEVIER CURRENT TRENDS, GB LNKD- DOI:10.1016/J.PT.2006.02.009, vol. 22, no. 4, 1 April 2006 (2006-04-01), pages 160-167, XP025229964 ISSN: 1471-4922 [retrieved on 2006-04-01]
- KARANIS, P. ET AL.: 'Development and preliminary evaluation of a loop-mediated isothermal amplification procedure for sensitive detection of Cryptosporidium oocysts in fecal and water samples' APPLIED AND ENVIRONMENTAL MICROBIOLOGY vol. 73, no. 17, September 2007, pages 5660 - 5662
- BAKHEIT, M.A. ET AL.: 'Sensitive and specific detection of Cryptosporidium species in PCR-negative samples by loop-mediated isothermal DNA amplification and confirmation of generated LAMP products by sequencing' VETERINARY PARASITOLOGY vol. 158, 2008, pages 11 - 22

### SEQUENCE LISTING

<110> Sydney water corporation, incorporated under the Sydney water Act, 1994 (NSW) Murdoch university
<120> A method of detecting microorganisms
<130> FBR 507621
<150> AU 2007906896
   <151> 2007-12-17
<160> 8
<170> PatentIn version 3.5
<210> 1
   <211> 21
   <212> DNA
   <213> Artificial sequence
<220>
   <223> Primer
<400> 1
   caagatgtgt tttcccatcg a 21
<210> 2
   <211> 20
   <212> DNA
   <213> Artificial Sequence
<220>
   <223> Primer
<400> 2
   cctctggagc aacacgtaat 20
<210> 3
   <211> 43
   <212> DNA
   <213> Artificial Sequence
<220>
   <223> Primer
<220>
   <221> mise_feature
   <222> (25)..(25)
   <223> n = linker or not present, n may be a number of nucleotides, e.g. TTTT
<400> 3
   ctttgattga gcttcatcac caacngccag gtgttatggt agg 43
<210> 4
   <211> 49
   <212> DNA
   <213> Artificial sequence
<220>
   <223> Primer
<220>
   <221> misc_feature
   <222> (25)..(25)
   <223> n = linker or not present, n may be a number of nucleotides, e.g. TTTT
<400> 4
   cttgaaatac ccaattgagc atggnttata gaaagtatga tgccagatc 49
<210> 5
   <211> 19
   <212> DNA
   <213> Artificial Sequence
<220>
   <223> Primer
<400> 5
   aggtgttatg gtaggtatg 19
<210> 6
   <211> 20
   <212> DNA
   <213> Artificial Sequence
<220>
   <223> Primer
<400> 6
   agaaagtatg atgccagatc 20
<210> 7
   <211> 42
   <212> DNA
   <213> Artificial Sequence
<220>
   <223> Primer
<400> 7
   ctttgattga gcttcatcac caacgccagg tgttatggta gg 42
<210> 8
   <211> 49
   <212> DNA
   <213> Artificial sequence
<220>
   <223> Primer
<400> 8
   cttgaaatac ccaattgagc atgggttata gaaagtatga tgccagatc 49

## Description

### FIELD OF THE INVENTION

The present invention relates to a method for the detection and/or identification of *Cryptosporidium* organisms in general, and one or more of *C. hominis, C. parvum* and *C*. *meleagridis* organisms in particular and/or nucleic acid sequences and to hybridization assay probes, helper probes, amplification primers, nucleic acid compositions, probe mixes, methods and kits useful for determining the presence of *Cryptosporidium* organisms in general, and one or more of *C. hominis, C. parvum* and *C. meleagridis* organisms in particular, in a test sample of water, faeces, food or other sample media.

### BACKGROUND OF THE INVENTION

*Cryptosporidium* spp. (phylum Apicomplexa) are coccidian protozoans capable of parasitizing the intestinal tract of a variety of mammalian species. *Cryptosporidium* spp. are prevalent in most vertebrate groups. Animals, such as cattle, sheep, etc may constitute an important reservoir of the human *Cryptosporidium* that infect humans. Disease outbreaks in day-care centres, hospitals and urban family groups, however, indicate that most human infections are transmitted person-to-person rather than via zoonotic or waterborne routes. Since oocysts are found almost exclusively in stool, the transmission is undoubtedly faecal-oral. Nevertheless, the occasional recovery of oocysts from both surface and drinking water suggests that indirect transmission via water is possible.

First recognized as a veterinary pathogen, its importance as a cause of human disease was not appreciated until the development of the acquired immunodeficiency syndrome epidemic. *C*. *parvum* and *C*. *hominis* in particular are now recognized as a significant cause of diarrhoeal disease in both immuno-suppressed and immunocompetent people worldwide. The disease has an incubation period of 1 - 14 days with symptoms lasting for 7 - 60 days. This intestinal pathogen has also been shown to play a significant role in the vicious diarrhoea/malnutrition cycle experienced by young children in developing countries. Resistance to chlorination and lack of an effective clinical treatment contribute to the organism's health impact.

*Cryptosporidium* spp. exist in nature in the form of environmentally resistant, thick walled oocysts. These oocysts have been known to survive for considerable periods in water and are unaffected by conventional water disinfectants, such as chlorine. After ingestion, motile sporozoites released from the oocysts by the action of bile acids within the gut lumen invade the epithelial cells lining the intestine, form parasitophorous vacuoles beneath the microvillus membranes of these host cells, and initiate a complex life cycle containing both sexual and asexual reproductive stages.

Known methods for detecting Cryptosporidium spp. include antibody staining methods referred to as *"Method 1622"* and *"Method 1623".* These methods have several drawbacks, including the subjectivity of the assay, the inability of the method to discriminate Cryptosporidium species, and its labour intensive aspects, requiring hours of microscopic analysis. Other methods either lack sensitivity or specificity or require expensive equipment and technical expertise to perform. Known methods include LAMP (eg. Karanis, Applied and environmental Microbidogy, 2007, p. 5660-5662)

Accordingly, a need exists for a sensitive and specific assay which can be used to determine the presence of *Cryptosporidium* organisms, and *C*. *hominis, C. parvum* and *C*. *meleagridis* organisms in particular, in a test sample. More particularly, however, of the known species of *Cryptosporidium; C. hominis, C. parvum* and *C*. *meleagridis* have clinical significance in humans. There is, accordingly a need for an effective method for specifically detecting *C*. *hominis, C. parvum* and *C*. *meleagridis* in a test sample of water, faeces, food or other sample media with respect to human health.

### SUMMARY OF THE INVENTION

According to the invention there is provided a method of detecting the presence of any one or more of *C*. *hominis, C. parvum* and *C*. *meleagridis* in a test sample, the method comprising contacting the test sample with:
(i) a first and a second outer primer, wherein the first outer primer is complementary to a region adjacent to the 5' terminus of a target DNA sequence and the second outer primer is complementary to a region adjacent to the 3' terminus of the target DNA sequence, and
(ii) a first and second inner primer, wherein the inner primers each contain two distinct sequences corresponding to a sense and antisense sequence of the target DNA sequence; and
performing auto-cycling strand displacement DNA synthesis, using a loop-mediated isothermal amplification ("LAMP") method and detecting the presence of amplified target DNA, wherein the Target DNA sequence is a conserved DNA sequence encoding action or parts thereof of *C*. *hominis, C. parvum and C. meleagridis.*

The detection may be qualitative, for example, detection may be performed using the dsDNA intercalating fluorescent dye SYTO-9, or quantitative, for example, using turbidity (Mori, 2004 J Biochem Biophys Methods, 59: 145-157) or intercalating dyes (Aoi, 2006 J Biotechnol, 125: 484-491).

The auto-cycling may be performed in the presence of a DNA polymerase.

The DNA polymerase may be a high strand displacement activity DNA polymerase.

The conserved target DNA sequence may encode actin.

The auto-cycling strand displacement DNA synthesis may comprise a heat-denaturing step, a reaction step and a termination step. The heat-denaturing step may occur for about 5 minutes at about 95°C. The reaction step may occur for about 35 minutes - 120 minutes at about 60°C - 66°C. The termination step may occur for about 2 minutes - 10 minutes at about 80°C.

The method may comprise contacting the amplified target DNA with a hybridization assay probe which preferentially hybridizes to the amplified target DNA, or a complement thereof, under stringent hybridization conditions, thereby forming a probe:amplified target DNA sequence, or complement thereof, hybrid stable for detection and detecting the presence of the probe:amplified target DNA sequence, or complement thereof, hybrid.

The invention also provides the use of an amplification primer for detecting the presence of any one or more of *C*. h*ominis, C. parvum* and *C*. *meleagridis* in an amplification assay, wherein The amplification primer comprises an at least 10 contiguous base region which is at least 80% identical (preferably at least 90% identical, and more preferably 100% identical) to an at least 10 contiguous base region present in any one of SEQ ID NOs 1 - 4

| | |
|---|---|
| caagatgtgttttcccatcga | SEQ ID NO: 1 |
| cctctggagcaacacgtaat | SEQ ID NO: 2 |
| ctttgattgagcttcatcaccaacngccaggtgttatggtagg | SEQ ID NO: 3 |
| cttgaaatacccaattgagcatggnttatagaaagtatgatgccagatc | SEQ ID NO: 4, |

wherein n may be a linker or not present. The linker may be a number of nucleotide bases, for example tttt.

The amplification primers of the present invention may be used in sets of at least two amplification primers. The invention additionally contemplates compositions comprising stable nucleic acid duplexes formed between the above-described amplification primers and the target DNA sequence for each primer under amplification conditions.

The description also provides a hybridization probe for determining whether any one or more of *C*. *hominis, C. parvum* and *C*. *meleagridis* is/are present in a test sample, which probes preferably contain an at least 10 contiguous base region which is at least 80% identical (preferably at least 90% identical, and more preferably 100% identical) to an at least 10 contiguous base region present in any one of SEQ ID NOs 1 - 4. These probes may preferentially hybridize to a target nucleic acid which is at least 80% complementary (preferably at least 90% complementary, and more preferably 100% complementary) any one or more of *C*. *hominis, C*. *parvum* and *C*. *meleagridis* and not to nucleic acid derived from non *C*. *hominis, C. parvum* and *C*. *meleagridis* under stringent hybridization assay conditions.

The probe may include a detectable label. The label may be any suitable labelling substance, including but not limited to a radioisotope, an enzyme, an enzyme cofactor, an enzyme substrate, a dye, a hapten, a chemiluminescent molecule, a fluorescent molecule, a phosphorescent molecule, an electrochemiluminescent molecule, a chromophore, a base sequence region that is unable to stably hybridize to the target nucleic acid.

The amplified target DNA may be detected by an electrophoretic method. The electrophoretic method may be a capillary electrophoretic method, a gel electrophoresis method. The amplified target DNA may be detected by Southern blotting or by sequencing. The detection of a positive LAMP reaction may be by visualisation of the end of the reaction. Due to the large amount of DNA produced by LAMP reactions, a white precipitate may be observed as a by-product. This precipitate is magnesium pyrophosphate. This precipitate may be related to an increase in turbidity which may be another means of detecting a positive reaction, for example with the use of a turbidometer. This may be either real-time measurement or end-point measurement. A further means of visualising the LAMP reaction result is by the addition of SYBR Green, where a positive reaction will turn a bright green upon mixing while a negative reaction will remain orange (Iwamoto, 2003, J Clin Microbiol, 41: 2616-2622).

Another means of detecting a positive LAMP reaction may be to use the addition of low molecular weight polyethylenimine to the reaction end-point. This may create an insoluble complex with any amplified DNA present. Labelled probes may be added in addition to the standard LAMP primers (Mori, 2006 BMC Biotechnol, 6: 3).

The description also provides a method of identifying at least one conserved nucleic acid sequence of *C*. *hominis, C. parvum* and *C*. *meleagridis* useful in the method of detecting the presence of a conserved target DNA sequence of any one or more of *C*. *hominis, C. parvum* and *C*. *meleagridis* in a test sample according to the invention. In one embodiment, the method comprises identifying at least two conserved nucleic acid sequences useful in the method of detecting the presence of a conserved target DNA sequence of any one or more of *C. hominis, C. parvum* and *C. meleagridis* in a test sample according to the invention. In one embodiment, the method comprises identifying at least three conserved nucleic acid sequences useful in the method of detecting the presence of a conserved target DNA sequence of any one or more of *C. hominis, C. parvum* and *C. meleagridis* in a test sample according to the invention. In one embodiment, the method comprises identifying at least four conserved nucleic acid sequences useful in the method of detecting the presence of a conserved target DNA sequence of any one or more of *C. hominis, C. parvum* and *C. meleagridis* in a test sample according to the invention. In another embodiment, the method comprises identifying at least one sequence complementary to a sequence selected from:

| | |
|---|---|
| caagatgtgttttcccatcga | SEQ ID NO: 1 |
| cctctggagcaacacgtaat | SEQ ID NO: 2 |
| ctttgattgagcttcatcaccaacngccaggtgttatggtagg | SEQ ID NO: 3 |
| cttgaaatacccaattgagcatggnttatagaaagtatgatgccagatc | SEQ ID NO: 4, |

wherein the at least one sequence complementary to a sequence selected from SEQ ID NOs: 1 - 4 is useful in the method of detecting the presence of a conserved target DNA sequence of any one or more of *C*. *hominis*, *C. parvum* and *C*. *meleagridis* in a test sample according to the invention. The conserved nucleic acid sequence(s) may encode actin or part(s) thereof.

n may be a linker or not present. The linker may be a number of nucleotide bases, for example tttt.

The description also provides a kit for determining whether any one or more of *C. hominis, C. parvum* and *C. meleagridis* is/are present in a test sample.

The kit may comprise at least one hybridization probe according to the invention and optionally include written instructions for determining the presence of any one or more of *C*. *hominis, C. parvum* and *C*. *meleagridis* is/are present in a test sample.

The kit may comprise at least one amplification probe according to the invention.

The kit may comprise at least one hybridization probe according to the invention and at least one amplification probe according to the invention. The invention also contemplates kits for amplifying the target DNA sequence according to the invention, comprising at least one of the amplification primers according to the invention and optionally include written instructions for amplifying nucleic acid. In a preferred embodiment, the kit will further comprise nucleotides and/or at least one enzyme required for amplification.

Those skilled in the art will appreciate that the hybridization assay probes of the present invention may be used as amplification primers or capture probes and the amplification primers of the present invention may be used as hybridization assay probes or capture probes, depending upon the degree of specificity required.

Throughout this specification the word *"comprise",* or variations such as *"comprises"* or *"comprising",* will be understood to imply the inclusion of a stated element, integer or step, or group of elements, integers or steps, but not the exclusion of any other element, integer or step, or group of elements, integers or steps.

The incorporation of these references, standing alone, should not be construed as an assertion or admission by the inventors that any portion of the contents of all of these references, or any particular reference, is considered to be essential material for satisfying any statutory disclosure requirement for patent applications.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

Other characteristics and advantages of the invention will appear through the description of non-limiting embodiments of the invention, which will be illustrated, with the help of the enclosed Figures among which:

### BRIEF DESCRIPTION OF THE FIGURES

- Figure 1:: The nucleotide sequences of *C*. *hominis, C. parvum* and *C*. *meleagridis* aligned with the LAMP primers (indicated by dashes with arrowheads at terminating bases).
- Figure 2:: Real-time amplification profile of LAMP reaction using SYTO-9 ds-DNA binding dye.
- Figure 3:: LAMP reactions by 1.5% agarose gel electrophoresis. 1. *C*. *hominis* 6149; 2. *C*. *parvum* IOWA; 3. C. *meleagridis* CZ-B1-32; 4. *C*. *baileyi* CZ-B1-15; 5. *C*. *andersoni* CZ-B1-52; 6. No DNA; M. 100 bp ladder.

### DETAILED DESCRIPTION OF PREFERRED EMBODIMENTS

The present invention provides an effective method for specifically detecting *C*. *parvum, C*. *hominis,* and *C*. *meleagridis* in a test sample of water, faeces, food or other sample media with respect to human health.

### Definitions

The following terms have the indicated meanings unless expressly indicated to have a different meaning:
*"Detecting the presence or absence"* is used in the sense of clinically acceptable standards for the presence or absence of *Cryptosporidium* spp.
*"Nucleic acid amplification."* or *"target amplification"* refers to increasing the number of nucleic acid molecules having at least one target nucleic acid sequence. Target amplification according to the present invention may be either linear or exponential, although exponential amplification is preferred.
*"Monitoring"* is used in the sense of checking or examining systematically for the presence or absence of *Cryptosporidium* spp.
*"Stringent hybridization assay conditions", "hybridization assay conditions" "stringent hybridization conditions"* or *"stringent conditions"* refers to conditions permitting a hybridization assay probe to preferentially hybridize to a target nucleic acid (a nucleic acid specific to *Cryptosporidium* spp. organisms) and not to nucleic acid derived from a closely related non-target microorganism. Stringent hybridization assay conditions may vary depending upon factors including the GC content and length of the probe, the degree of similarity between the probe sequence and sequences of non-target sequences which may be present in the test sample, and the target sequence. Hybridization conditions include the temperature and the composition of the hybridization reagents or solutions.
*"Target nucleic acid sequence", "target nucleotide sequence", "target sequence"* or *"target region"* refers to a specific deoxyribonucleotide or ribonucleotide sequence comprising all or part of the nucleotide sequence of a single-stranded nucleic acid molecule.
*"Target nucleic acid"* or *"target"* refers to a nucleic acid containing a target nucleic acid sequence.

The present invention features isolated nucleic acid molecules which are useful for determining whether organisms belonging to the genus *Cryptosporidium* spp. are present in a test sample such as water, faeces, food or other sample media, specifically the presence of any one or more of *C hominis, C. parvum* and *C. meleagridis* in a test sample of water, faeces, food or other sample media with respect to human health.

### Method of detecting the presence of any one or more of C. hominis. C. parvum and C. meleagridis in a test sample

According to the invention there is provided a method of detecting the presence of any one or more of *C. hominis, C. parvum* and *C. meleagridis* in a test sample, the method comprising contacting the test sample with:
(i) a first and a second outer primer, wherein the first outer primer is complementary to a region adjacent to the 5' terminus of a target DNA sequence and the second outer primer is complementary to a region adjacent to the 3' terminus of the target DNA sequence, and
(ii) a first and second inner primer, wherein the inner primers each contain two distinct sequences corresponding to a sense and antisense sequence of the target DNA sequence; and
performing auto-cycling strand displacement DNA synthesis, and detecting the presence of amplified target DNA, wherein the target DNA sequence is a substantially conserved DNA sequence of *C*. *hominis, C. parvum* and *C*. *meleagridis.*

The detection may be qualitative, for example, detection may be performed using the dsDNA intercalating fluorescent dye SYTO-9, or quantitative, for example, using turbidity (Mori, 2004 J Biochem Biophys Methods, 59: 145-157) or intercalating dyes (Aoi, 2006 J Biotechnol, 125: 484-491).

The auto-cycling may be performed in the presence of a DNA polymerase.

The DNA polymerase may be a high strand displacement activity DNA polymerase.

The conserved target DNA sequence(s) may encode(s) actin or part(s) thereof.

The auto-cycling strand displacement DNA synthesis may be a loop-mediated isothermal amplification *("LAMP")* method.

The auto-cycling strand displacement DNA synthesis may comprise a heat-denaturing step, a reaction step and a termination step. The heat-denaturing step may occur for about 5 minutes at about 95°C. The reaction step may occur for about 35 minutes - 120 minutes at about 60°C - 66°C. The termination step may occur for about 2 minutes -10 minutes at about 80°C.

The method may comprise contacting the amplified target DNA with a hybridization assay probe which preferentially hybridizes to the amplified target DNA, or a complement thereof, under stringent hybridization conditions, thereby forming a probe:amplified target DNA sequence, or complement thereof, hybrid stable for detection and detecting the presence of the probe:amplified target DNA sequence, or complement thereof, hybrid.

### Amplification primers

The invention also provides an amplification primer useful for detecting the presence of any one or more of *C*. *hominis, C. parvum* and *C*. *meleagridis* in an amplification assay. The amplification primer may contain an at least 10 contiguous base region which is at least 80% identical (preferably at least 90% identical, and more preferably 100% identical) to an at least 10 contiguous base region present in any one of SEQ ID NOs 1 - 4

| | |
|---|---|
| caagatgtgttttcccatcga | SEQ ID NO: 1 |
| cctctggagcaacacgtaat | SEQ ID NO: 2 |
| ctttgattgagcttcatcaccaacngccaggtgttatggtagg | SEQ ID NO: 3 |
| cttgaaatacccaattgagcatggnttatagaaagtatgatgccagatc | SEQ ID NO: 4. |

n may be a linker or not present. The linker may be a number of nucleotide bases, for example tttt.

The amplification primers of the present invention may be used in sets of at least two amplification primers. The invention additionally contemplates compositions comprising stable nucleic acid duplexes formed between the above-described amplification primers and the target DNA sequence for each primer under amplification conditions.

The amplification primer may contain an at least 10 contiguous base region which is at least 80% homologous (preferably at least 90% homologous, and more preferably 100% homologous) to an at least 10 contiguous base region present in any one of SEQ ID NOs 1 - 4. The amplification primers of the present invention may be used in sets of at least two amplification primers. The invention additionally contemplates compositions comprising stable nucleic acid duplexes formed between the above-described amplification primers and a target nucleic acid for each primers under amplification conditions. If the amplification primer is designed to initiate RNA synthesis, the primer may contain a base sequence which is non-complementary to the target sequence but which is recognized by an RNA polymerase such as a T7, T3 or SP6 RNA polymerase. An amplification primer of the invention may contain a 3' terminus which is modified to prevent or lessen the rate or amount of primer extension. (McDonough *et al.,* "Methods of Amplifying Nucleic Acids Using Promoter-Containing Primer Sequence," U.S. Pat. No. 5,766,849, disclose primers and promoter-primers having modified or blocked 3'-ends). While the amplification primers of the present invention may be chemically synthesized or derived from a vector, they are not naturally-occurring nucleic acid molecules.

The amplification primers of the invention may be applied to crude preparations of oocysts, faecal or water samples, together with LAMP reaction reagents and the performance of the reaction is assessed under these conditions. Oocysts may be isolated by concentration from water for releasing DNA according to isolation methods well known to those of ordinary skill in the art. Amplification using LAMP can then be used to detect the presence of any one or more of *C*. *hominis, C. parvum* and C. *meleagridis.* Loop-Mediated Isothermal Amplification ("*LAM*P") is a DNA amplification method which does not require thermocycling (Notomi *et al.,* 2000). It utilizes a DNA polymerase with strand displacement activity as well as a set of at least four primers specific for six regions on the target DNA. The highly sensitive and specific LAMP reaction may be performed under isothermal conditions (for example, 60°C - 65°C) in usually less than one hour.

### Detectable probes

The invention also provides a detectable probe for determining whether any one or more of *C. hominis, C. parvum* and *C. meleagridis* is/are present in a test sample, which probes preferably contain an at least 10 contiguous base region which is at least 80% identical (preferably at least 90% identical, and more preferably 100% identical) to an at least 10 contiguous base region present in any one of SEQ ID NOs 1 - 4. These probes may preferentially hybridize to a target nucleic acid which is at least 80% complementary (preferably at least 90% complementary, and more preferably 100% complementary) any one or more of *C. hominis, C. parvum* and *C. meleagridis* and not to nucleic acid derived from non *C. hominis, C. parvum* and *C. meleagridis* under stringent hybridization assay conditions.

The probe may include a detectable label. The label may be any suitable labelling substance, including but not limited to a radioisotope, an enzyme, an enzyme cofactor, an enzyme substrate, a dye, a hapten, a chemiluminescent molecule, a fluorescent molecule, a phosphorescent molecule, an electrochemiluminescent molecule, a chromophore, a base sequence region that is unable to stably hybridize to the target nucleic acid.

The detectable probes of the present invention may include one or more base sequences in addition to the base sequence of the target binding region which do not stably bind to nucleic acid derived from any one or more of *C*. *hominis, C. parvum* and *C. meleagridis,* under stringent conditions. An additional base sequence may be comprised of any desired base sequence, so long as it does not stably bind to nucleic acid derived from the target organism under stringent conditions or prevent stable hybridization of the probe to the target nucleic acid. By way of example, an additional base sequence may constitute the immobilized probe binding region of a capture probe, where the immobilized probe binding region is comprised of, for example, a 3' poly dA (adenine) region which hybridizes under stringent conditions to a 5' poly dT (thymine) region of a polynucleotide bound directly or indirectly to a solid support. An additional base sequence might also be a 5' sequence recognized by an RNA polymerase or which enhances initiation or elongation by an RNA polymerase (e.g., a T7 promoter). More than one additional base sequence may be included if the first sequence is incorporated into, for example, a *"molecular beacon"* probe. Molecular beacons are disclosed by Tyagi *et al.,* "Detectably Labeled Dual Conformation Oligonucleotide Probes, Assays and Kits," U.S. Pat. No. 5,925,517, and include a target binding region which is bounded by two base sequences having regions which are at least partially complementary to each other. An additional base sequence may be joined directly to the target binding region or, for example, by means of a non-nucleotide linker.

The detectable probe of the invention may comprise a base sequence suffciently complementary to its target nucleic acid sequence to form a probe:target hybrid stable for detection under stringent hybridization assay conditions. As would be understood by someone having ordinary skill in the art, a hybridization probe is an isolated nucleic acid molecule, or an analog thereof, in a form not found in nature without human intervention (e.g., recombined with foreign nucleic acid, isolated, or purified to some extent).

The detectable probe according to the invention may comprise additional nucleosides or nucleobases outside of the targeted region so long as such nucleosides or nucleobases do not prevent hybridization under stringent hybridization conditions and, in the case of hybridization assay probes, do not prevent preferential hybridization to the target nucleic acid. A non-complementary sequence may also be included, such as a target capture sequence (generally a homopolymer tract, such as a poly-A, poly-T or poly-U tail), promotor sequence, a binding site for RNA transcription, a restriction endonuclease recognition site, or sequences which will confer a desired secondary or tertiary structure, such as a catalytic active site or a hairpin structure, which can be used to facilitate detection and/or amplification. A detectable probe according to the invention may be produced by techniques known to those of ordinary skill in the art, such as by chemical synthesis, and by *in vitro* or *in vivo* expression from recombinant nucleic acid molecules.

A person skilled in the art will appreciate that hybridization refers to the ability of two completely or partially complementary nucleic acid strands to come together under specified hybridization assay conditions in a parallel or preferably antiparallel orientation to form a stable structure having a double-stranded region. The two constituent strands of this double-stranded structure, sometimes called a hybrid, are held together by hydrogen bonds. Although these hydrogen bonds most commonly form between nucleotides containing the bases adenine and thymine or uracil (A and T or U) or cytosine and guanine (C and G) on single nucleic acid strands, base pairing can also form between bases which are not members of these *"canonical"* pairs. Non-canonical base pairing is well-known in the art. (See, e.g., Roger L. P. Adams et al., The Biochemistry of the Nucleic Acids (11th ed 1992)).

While not required, the probes preferably include a detectable label or group of interacting labels. The label may be any suitable labelling substance, including but not limited to a radioisotope, an enzyme, an enzyme cofactor, an enzyme substrate, a dye, a hapten, a chemiluminescent molecule, a fluorescent molecule, a phosphorescent molecule, an electrochemiluminescent molecule, a chromophore, a base sequence region that is unable to stably bind to the target nucleic acid under the stated conditions, and mixtures of these. In one particularly preferred embodiment, the label is an acridinium ester (AE), preferably 4-(2-succinimidyloxycarbonyl ethyl)-phenyl-10-methylacridinium-9-carboxylate fluorosulfonate (hereinafter referred to as *"standard AE*")*.* Groups of interacting labels include, but are not limited to, enzyme/substrate, enzyme/cofactor, luminescent/quencher, luminescent/adduct, dye dimers and Forrester energy transfer pairs.

### Capture probes

A person skilled in the art will be aware that a *"capture probe"* is an oligonucleotide or a set of at least two oligonucleotides linked together which are capable of hybridizing to a target nucleic acid and to an immobilized probe, thereby providing means for immobilizing and isolating the target nucleic acid in a test sample. That portion of the capture probe which hybridizes to the target nucleic acid is referred to as the *"target binding region",* and that portion of the capture probe which hybridizes to the immobilized probe is referred to as the *"immobilized probe binding region".* While the preferred capture probe hybridizes to both the target nucleic acid and the immobilized probe under assay conditions, the target binding region and the immobilized probe binding region may be designed to hybridize to their respective target sequences under different hybridization conditions. In this way, the capture probe may be designed so that it first hybridizes to the target nucleic acid under more favourable in solution kinetics before adjusting the conditions to permit hybridization of the immobilized probe binding region to the immobilized probe. When the target binding and immobilized probe binding regions are provided on the same capture probe, they may be directly adjoining each other on the same oligonucleotide, they may be separated from each other by one or more optionally modified nucleotides, or they may be joined to each other by means of a non-nucleotide linker. The *"target binding region"* is that portion of an oligonucleotide which stably binds to a target sequence present in a target nucleic acid, a DNA or RNA equivalent of the target sequence or a complement of the target sequence under assay conditions. The assay conditions may be stringent hybridization conditions or amplification conditions.

The *"immobilized probe binding region"* is herein understood to be that portion of an oligonucleotide which hybridizes to an immobilized probe under assay conditions.

The *"immobilized probe"* is herein understood to be an oligonucleotide for joining a capture probe to an immobilized support. The immobilized probe is joined either directly or indirectly to the solid support by a linkage or interaction which remains stable under the conditions employed to hybridize the capture probe to the target nucleic acid and to the immobilized probe, whether those conditions are the same or different. The immobilized probe facilitates separation of the bound target nucleic acid from unbound materials in a sample.

The present invention provides a capture probe comprising at least one oligonucleotide containing an immobilized probe binding region and a target binding region. The immobilized probe binding region of the capture probes may be comprised of any base sequence capable of stably hybridizing under stringent conditions to oligonucleotides bound to a solid support present in a test sample. The immobilized probe binding region may be a poly dA, homopolymer tail located at the 3' end of the capture probe. The oligonucleotides bound to the solid support may include 5' poly dT tails of sufficient length to stably bind to the poly dA tails of the capture probes under assay conditions. The immobilized probe binding region may include a poly dA tail which is about 30 adenines in length, and the capture probe includes a spacer region which is about 3 thymines in length for joining target binding region and the immobilized probe binding region to each other.

The target binding region of the capture probes may stably bind to a target sequence present in nucleic acid derived from any one or more of *C. hominis, C. parvum* and *C. meleagridis.*

The target binding region of the capture probe may comprise a base sequence region which is at least about 85% complementary (preferably at least about 90% complementary, more preferably at least about 95% complementary, and most preferably 100% complementary) to a base sequence selected from the group consisting of SEQ ID NOs 1 - 4.

To prevent undesirable cross-hybridization reactions, the capture probe may exclude nucleotide base sequences, other than the nucleotide base sequence of the target binding region, which can stably bind to nucleic acid derived from any organism which may be present in the test sample under assay conditions. Consistent with this approach, and in order to maximize the immobilization of capture probe:target complexes which are formed, the nucleotide base sequence of the immobilized probe binding region may be designed so that it can stably bind to a nucleotide base sequence present in the immobilized probe under assay conditions and not to nucleic acid derived from any non *C. hominis, C. parvum* and *C. meleagridis* organism which may be present in the test sample.

The target binding region and the immobilized probe binding region of the capture probe may be selected so that the capture probe:target complex has a higher Tₘ than the Tₘ of the capture probe:immobilized probe complex. In this way, a first set of conditions may be imposed which favours hybridization of the capture probe to the target sequence over the immobilized probe, thereby providing for optimal liquid phase hybridization kinetics for hybridization of the capture probe to the target sequence. Once sufficient time has passed for the capture probe to bind to the target sequence, a second set of less stringent conditions may be imposed which allows for hybridization of the capture probe to the immobilized probe.

Capture probes of the present invention may also include a label or a pair of interacting labels for direct detection of the target sequence in a test sample. Non-limiting examples of labels, combinations of labels and means for labelling probes are known to persons skilled in the art.

The immobilized probe may be joined to a magnetically charged particle which can be isolated in a reaction vessel during a purification step once the probe has had sufficient time to hybridize to target nucleic acid present in the sample. (Acosta *et al.,* "Assay Work Station," U.S. Pat. No. 6,254,826, disclose an instrument for performing such a purification step.) The capture probe may be designed so that the melting temperature of the capture probe:target hybrid is greater than the melting temperature of the capture probe:immobilized probe hybrid. In this way, different sets of hybridization assay conditions can be employed to facilitate hybridization of the capture probe to the target nucleic acid prior to hybridization of the capture probe to the immobilized oligonucleotide, thereby maximizing the concentration of free probe and providing favourable liquid phase hybridization kinetics. This *"two-step"* target capture method is disclosed by Weisburg et al., U.S. Pat. No. 6,110,678. Other target capture schemes which could be readily adapted to the present invention are well known in the art and include, without limitation, those disclosed by the following: Dunn et al., Methods in Enzymology, "Mapping viral mRNAs by sandwich hybridization," 65(1):468 478 (1980); Ranki et al., U.S. Pat. No. 4,486,539; Stabinsky, U.S. Pat. No. 4,751,177; and Becker et al., U.S. Pat. No. 6,130,038.

### Detectable labels and detection methods

The probe/primer may include a detectable label. The label may be any suitable labelling substance, including but not limited to a radioisotope, an enzyme, an enzyme cofactor, an enzyme substrate, a dye, a hapten, a chemiluminescent molecule, a fluorescent molecule, a phosphorescent molecule, an electrochemiluminescent molecule, a chromophore, a base sequence region that is unable to stably hybridize to the target nucleic acid. Probing can be carried out by using radiolabeled probes in Southern Blotting, but use of other probing techniques is also provided, e.g., by tagging oligonucleotides complementary to the target sequence with a marker, such as biotin or a fluorescent agent, and relating presence of bound marker with a positive result, or by using biotin tag as active agent in an ELISA in the known manner or detecting fluorescence.

The invention provides an *in situ* detection method that is a direct technique, which involves incorporation of a label directly into the amplification product. For example, a reporter molecule such as digoxigenin [DIG]-dUTP or fluorescein-dUTP may be included in the amplification cocktail and incorporated into the amplification product. A simple immunochemical step using alkaline phosphatase- or peroxidase-conjugated anti-DIG then detects DIG labelled amplification products. Alternatively, fluorescein labelled amplification products can be detected by epifluorescence microscopy or immunochemical methods.

The invention also provides an indirect technique for detection, which involves hybridization of a specific labelled probe to the amplification product after PCR. The label on the probe may then be detected either by, for example, immunochemical methods or epifluorescence microscopy.

The amplified target DNA may be detected by an electrophoretic method. The electrophoretic method may be a capillary electrophoretic method, a gel electrophoresis method. The amplified target DNA may be detected by Southern blotting or by sequencing. The detection of a positive LAMP reaction may be by visualisation of the end of the reaction. Due to the large amount of DNA produced by LAMP reactions, a white precipitate may be observed as a by-product. This precipitate is magnesium pyrophosphate. This precipitate may be related to an increase in turbidity which may be another means of detecting a positive reaction, for example with the use of a turbidometer. This may be either real-time measurement or end-point measurement. A further means of visualising the LAMP reaction result is by the addition of SYBR Green, where a positive reaction will turn a bright green upon mixing while a negative reaction will remain orange (Iwamoto, 2003, J Clin Microbiol, 41: 2616-2622).

Another means of detecting a positive LAMP reaction may be to use the addition of low molecular weight polyethylenimine to the reaction end-point. This may create an insoluble complex with any amplified DNA present. Labelled probes may be added in addition to the standard LAMP primers (Mori, 2006 BMC Biotechnol, 6: 3).

Amplified DNA may be detected directly by any method that can distinguish among different lengths of DNA. Electrophoresis through agarose is the standard method used to separate, identify, and purify DNA fragments. The location of DNA within the gel can be determined directly by staining with low concentrations of the fluorescent intercalating dye ethidium bromide. Bands corresponding to the predicted length for amplified target DNA can then be detected by direct examination of the gel in ultraviolet light.

In addition, the DNA bands from an electrophoresed gel can be transferred to a membrane support by capillary action, followed by indirect detection using oligonucleotide probes. A typical transfer protocol includes denaturing the DNA within the gel using an alkaline solution, such as 0.4M NaOH, 0.6M NaCl, followed by a neutralization step in a buffer solution, e.g. 1.5M NaCl, 0.5M Tris-HCl, pH 7.5. The gel is then equilibrated with a high ionic strength transfer buffer, such as 1X SSC, wherein 1X SSC is 0.15M NaCl, 0.015M Na citrate. The denatured DNA can then be transferred from the gel to a membrane support by capillary blotting in transfer buffer.

Amplified DNA, that has been captured on a solid support, such as nylon or nitrocellulose membrane, may be detected by using a labelled hybridization probe. The probe may be labelled with a radioactive or fluorescent tag, or attached directly or indirectly to an enzyme molecule. Then, the membrane-bound amplified target DNA is incubated with the probe under hybridization conditions. Following hybridization, excess probe is washed away. If the hybridization probe is radioactively tagged, the remaining hybridized probe can be detected by autoradiography or scintillation counting. If the probe contains biotin or some other chemical group for which there are specific binding molecules, like avidin and antibodies, then the immobilized probe can be detected with an enzyme attached to the specific binding molecule, such as horseradish peroxidase or alkaline phosphatase attached to streptavidin.

Detection may be via hybridization with a non-radioactive 5'digoxigenin (DIG)-labeled oligonucleotide probe. Following hybridization the solid support is washed with a high ionic strength buffer, such as 5X SSC, at about 70°C. The immobilized hybridization probe that remains after washing can be visualized by incubating the solid support with anti-DIG antibody conjugated to alkaline phosphatase, followed by addition of a chemiluminescent substrate, such as Lumigen-PPD (Boehringer Mannheim). The support is finally washed, sealed in plastic wrap, and exposed to X-ray film to detect any chemiluminescence.

### Methods of identifying at least one conserved nucleic acid sequence of C. hominis, C parvum and C. meleagridis

The invention also provides a method of identifying at least one conserved nucleic acid sequence of *C. hominis, C. parvum* and *C. meleagridis* useful in the method of detecting the presence of a conserved target DNA sequence of any one or more of C. *hominis, C. parvum* and *C. meleagridis* in a test sample according to the invention. In one embodiment, the method comprises identifying at least two conserved nucleic acid sequences useful in the method of detecting the presence of a conserved target DNA sequence of any one or more of *C. hominis, C. parvum* and *C. meleagridis* in a test sample according to the invention. In one embodiment, the method comprises identifying at least three conserved nucleic acid sequences useful in the method of detecting the presence of a conserved target DNA sequence of any one or more of *C. hominis, C. parvum* and *C. neeleagridis* in a test sample according to the invention. In one embodiment, the method comprises identifying at least four conserved nucleic acid sequences useful in the method of detecting the presence of a conserved target DNA sequence of any one or more of *C. hominis, C. parvum* and *C. meleagridis* in a test sample according to the invention. In another embodiment, the method comprises identifying at least one sequence complementary to a sequence selected from:

| | |
|---|---|
| caagatgtgttttcccatcga | SEQ ID NO: 1 |
| cctctggagcaacacgtaat | SEQ ID NO: 2 |
| ctttgattgagcttcatcaccaacngccaggtgttatggtagg | SEQ ID NO: 3 |
| cttgaaatacccaattgagcatggnttatagaaagtatgatgccagatc | SEQ ID NO: 4, |

wherein the at least one sequence complementary to a sequence selected SEQ ID NOs: 1- 4 is useful in the method of detecting the presence of a conserved target DNA sequence of any one or more of *C. hominis, C. parvum* and *C. meleagridis* in a test sample according to the invention, and wherein n may be a linker or not present The linker may be a number of nucleotide bases, for example tttt. The conserved nucleic acid sequence(s) may encode actin or part(s) thereof.

The method may comprise a step of contacting a test sample with at least one helper probe, as desired. See Hogan *et al.,* "Means and Method for Enhancing Nucleic Acid Hybridization," U.S. Pat. No. 5,030,557. A "*helper probe*" is herein understood to be an oligonucleotide designed to hybridize to a target nucleic acid at a different locus than that of a hybridization assay probe, thereby either increasing the rate of hybridization of the probe to the target nucleic acid, increasing the melting temperature of the probe:target hybrid, or both.

### Kits

The primers and/or probes of the invention, used to amplify and detect any one or more of *C. hominis, C. parvum* and *C. meleagridis* organisms, can be conveniently packaged as kits. The kit may comprise suitable amounts of primer, or a suitable amount of a probe, or suitable amounts of a primer and probe according to the invention. In addition, kits can contain a suitable amount of at least one standard sample, which contains a known concentration of a *Cryptosporidium* species, and a negative control sample substantially free of the protozoa of interest.

The methods and kits of the present invention have many advantages over previous methods, including the speed, sensitivity, and specificity associated with amplification procedures, such as PCR. Moreover, *C. hominis, C. parvum* and *C. meleagridis* can be distinguished from other Cryptosporidia, which only infect non-human animal hosts.

The kit may comprise at least one hybridization probe according to the invention and optionally include written instructions for determining the presence of any one or more of *C. hominis, C. parvum* and *C. meleagridis* in a test sample.

The kit may comprise at least one amplification probe according to the invention.

The kit may comprise at least one hybridization probe according to the invention and at least one amplification probe according to the invention. The invention also contemplates kits for amplifying the target DNA sequence according to the invention, comprising at least one of the amplification primers according to the invention and optionally include written instructions for amplifying nucleic acid. In a preferred embodiment, the kit will further comprise nucleotides and/or at least one enzyme required for amplification.

The primers and/or probes according to the invention may be used to detect any one or more of *C. hominis, C. parvum* and *C. meleagridis* in oocyst form or otherwise, and can be conveniently packaged as kits. The kit may comprise suitable amounts of the primers, or a suitable amount of the probe, or suitable amounts of the primers and probe. In addition, kits can contain a suitable amount of at least one standard sample, which contains a known concentration of any one or more of *C. hominis, C. parvum* and *C. meleagridis* in oocyst form or otherwise.

The kit may comprise at least one hybridization probe according to the invention and optionally include written instructions for determining the presence or amount of one or more of *C. hominis, C. parvum* and *C. meleagridis* in a test sample.

In another embodiment, the kit may comprise at least one amplification probe according to the invention.

The kit may comprise at least one hybridization probe according to the invention and at least one amplification probe according to the invention.

The kit may comprise, in addition to a hybridization assay probe, at least one amplification primer according to the invention.

The kit may further comprise, in addition to the hybridization assay probes, at least one capture probe according to the invention.

The kit may comprise, in addition to the hybridization assay probes, at least one of the above-described capture probes and at least one of the above-described amplification primers.

The kit including a capture probe may further include a solid support material (e.g., magnetically responsive particles) for immobilizing the capture probe, either directly or indirectly, in a test sample.

The kit may comprise required enzymes, the nucleotide triphosphates, and the probes and/or primers according to the invention. The nucleotide triphosphates, and the probes and/or primers according to the invention may be provided as a lyophilized reagent. The lyophilized reagents may be pre-mixed before lyophilization so that when reconstituted they form a complete mixture with the proper ratio of each of the components ready for use in the assay. In addition, the kit may contain a reconstitution reagent for reconstituting the lyophilized reagents of the kit.

Typical packaging materials would include solid matrices such as glass, plastic, paper, foil, micro-particles and the like, capable of holding within fixed limits hybridization assay probes, capture probes, helper probes and/or amplification primers of the present invention. Thus, for example, the packaging materials can include glass vials used to contain sub-milligram (e.g., picogram or nanogram) quantities of a contemplated probe or primer, or they can be microtiter plate wells to which probes or primers of the present invention have been operatively affixed, i.e., linked so as to be capable of participating in an amplification and/or detection method of the present invention.

The instructions will typically indicate the reagents and/or concentrations of reagents and at least one assay method parameter which might be, for example, the relative amounts of reagents to use per amount of sample. In addition, such specifics as maintenance, time periods, temperature and buffer conditions may also be included.

The kits may comprise a hybridization assay probe, a capture probe and/or amplification primer described herein, whether provided individually or in one of the preferred combinations described above, for use in amplifying and/or determining the presence or amount of any one or more of *C. hominis, C. parvum* and *C. meleagridis* in a test sample.

### Cross-functionality of probes and primers

Those skilled in the art will appreciate that the hybridization assay probes of the present invention may be used as amplification primers or capture probes and the amplification primers of the present invention may be used as hybridization assay probes or capture probes, depending upon the degree of specificity required.

The probe and/or primer may be DNA, a corresponding RNA and/or analogs thereof. The sugar groups of the nucleoside subunits may be ribose, deoxyribose and analogs thereof, including, for example, ribonucleosides having a 2'-O-methyl substitution to the ribofuranosyl moiety. (Oligonucleotides including nucleoside subunits having 2' substitutions and which are useful as hybridization assay probes, helper probes and/or amplification primers are disclosed by Becker *et al.,* "Method for Amplifying Target Nucleic Acids Using Modified Primers," U.S. Pat. No. 6,130,038.) The nucleoside subunits may by joined by linkages such as phosphodiester linkages, modified linkages or by non-nucleotide moieties which do not prevent hybridization of the oligonucleotide to its complementary target nucleic acid sequence. Modified linkages include those linkages in which a standard phosphodiester linkage is replaced with a different linkage, such as a phosphorothioate linkage or a methylphosphonate linkage. The nucleobase subunits may be joined, for example, by replacing the natural deoxyribose phosphate backbone of DNA with a pseuodo peptide backbone, such as a 2-aminoethylglycine backbone which couples the nucleobase subunits by means of a carboxymethyl linker to the central secondary amine. (DNA analogs having a pseudo peptide backbone are commonly referred to as "peptide nucleic acids" or "PNA" and are disclosed by Nielsen *et al.,* "Peptide Nucleic Acids," U.S. Pat. No. 5,539,082.) Other non-limiting examples of nucleic acid molecules contemplated by the present invention include nucleic acid analogs containing bicyclic and tricyclic nucleoside and nucleotide analogs referred to as "Locked Nucleic Acids," "Locked Nucleoside Analogues" or "LNA." (Locked Nucleic Acids are disclosed by Wang, "Conformationally Locked Nucleosides and Oligonucleotide," U.S. Pat. No. 6,083,482; Imanishi *et al.,* "Novel Bicyclonucleoside and Oligonucleotide Analogues," International Publication No. WO 98/39352; and Wengel *et al.,* "Oligonucleotide Analogues," International Publication No. WO 99/14226.) Any nucleic acid analog is contemplated by the present invention provided the modified oligonucleotide can hybridize to a target nucleic acid under stringent hybridization assay conditions or amplification conditions. In the case of hybridization assay probes, the modified oligonucleotides must also be capable of preferentially hybridizing to the target nucleic acid under stringent hybridization assay conditions.

### Liberating nucleic acid molecules in the test sample

It will be understood by those skilled in the art that when any of the methods outlined above are being applied to whole organisms, rather than to nucleic acids derived from them, it will be necessary to incorporate a procedure for liberating nucleic acids from said organism before initiating amplification chain reaction conditions. The preferred forms of the present invention provide particular procedures whereby the sample to be tested is first suspended in a medium, e.g., a buffer, and then either sonicated or subjected to freeze/thaw cycles in buffer containing a reducing agent, e.g., dithiothreitol (DTT), in order to rupture the organisms, particularly their oocyst forms, and liberate the nucleic acid.

Using these preferred methods of the present invention, it is possible to achieve detection or identification of any one or more of C. *hominis, C. parvum* and C. *meleagridis* in oocyst form or otherwise.

The sonication or freeze/thaw procedure may be carried out in a medium which will form all or part of the LAMP or probing medium. Sonication may conveniently be carried out using about 11 µm peak to peak. Alternatively, for freeze/thaw procedure, sample containers can be conveniently immersed in a cryogenic liquid such as liquid nitrogen. Other methods for disrupting cell or oocyst structures to liberate nucleic acids into the LAMP or probing medium will be known to the skilled person and may be expected to be applicable before initiating LAMP and/or probing procedures.

Subsequent hybridization and amplification procedures can also be performed using nucleic acid containing extracts from cysts, oocysts, and/or infected cell cultures. If the extracts are to be used for detecting DNA, RNA can be removed from the lysate by treatment with DNase-free RNase A. Further purification of DNA from oocysts and infected cell cultures can be accomplished by additional extraction steps. For example, cells can be lysed in 50 mM Tris-HCl, 20 mM EDTA, pH 8, containing 2 mg/ml proteinase K and 0.5% sarkosyl, and incubated at 37.degree °C for 1 h. Then, 5M NaCl is added to give a final concentration of 1M, and CTAB (hexadecyltrimethyl ammonium bromide) is added to a concentration of 1%. Following incubation at 65 °C for 30 min, the lysate is subjected to at least one freeze/thaw cycle, and phenol/chloroform extraction. The DNA is precipitated by the addition of 0.6 vol, of isopropanol and the DNA precipitate is then washed with 70% ethanol. A person skilled in the art will also appreciate that DNA/RNA extraction kits, for example, the Epicentre MasterPure^{™} and WaterMaster^{™} Kits (EPICENTRE Biotechnologies) may also be used to liberate nucleic acids from an organism before initiating amplification chain reaction conditions.

If the extracts are to be used for detecting RNA, then DNA can be removed from the lysate by treatment with RNase-free DNase. Total RNA can also be isolated from lysed cells by extraction with strong denaturants, such as guanidium thiocyanate, followed by centrifugation through a cesium chloride solution. Moreover, mRNA can be isolated using solid state particles attached to oligo-dT, which can select mRNA transcripts having a poly(A) tail.

### Recovery of oocysts

The diagnosis of *Cryptosporidium* spp. infection may generally be established by the recovery of *Cryptosporidium* oocysts from stool specimens. Alternatively, assessment of the risk of or evidence for the transmission of cryptosporidiosis via contaminated water may be provided by concentrating *Cryptosporidium* oocysts from water samples.

*Cryplosporidium* oocysts may be concentrated from water by a variety of methods. For example, a predetermined volume of water, e.g. 100 litres, may be filtered through a 1 µ nominal porosity yarn-wound polypropylene filter or its equivalent, with the filtration flow rate restricted to about 4 litres/min. Sampled filters may typically be shipped on ice to analytical laboratories for analysis within 24 hours. Retained protozoa may be eluted from the filter within 96 hours of collection with a buffered detergent solution, filter fibres are cut, teased and washed by hand or with the aid of a stomacher. Oocysts recovered in the eluent may be concentrated by centrifugation and partially purified by flotation on a Percoll-sucrose solution with a specific gravity of 1.1. A portion of the purified material may be placed on a membrane filter, tagged with antibody using the indirect staining method, and examined under epifluorescence microscopy. Specific criteria may be used to identify oocysts including, immunofluorescence, size, shape, and internal morphology.

### Flatbed membrane filtration method

This method was derived from that originally described by Ongerth and Stibbs (Appl. Environ. Microbiol. 53: 672 - 676, 1987). Water samples may be concentrated through a 293-mm-diameter flatbed filter unit (Millipore) containing a 2-µm pore size track-etched polycarbonate membrane (Osmonics, Massachusetts) using a peristaltic water pump (Watson Marlow unit 604S) at a rate not exceeding 4 L/min. The membrane may then be transferred to a modified perspex sheet (Micronix, Sydney, Australia), sprayed with elution buffer No. 1, and the concentrate eluted using a rubber squeegee (Micronix) and collected in a 50-mL centrifuge tube (Iwaki). This elution procedure may be repeated 2 to 4 times, following which the membrane may be removed and discarded and the perspex sheet and squeegee further rinsed with elution buffer. The filter wash may then be centrifuged at 1620g for 10 minutes at 4 ± 2 °C. The supernatant may be aspirated to leave the pellet remaining in a volume of 5 mL, which may then be resuspended by vortex mixing.

The flatbed apparatus may be cleaned between samples by backflushing with a detergent solution (Tergazyme, Alconox Inc., White Plains, New York) followed by water. The perspex screen and the squeegee may be scrubbed with detergent and rinsed out with water.

After concentration, samples may be processed using immunomagnetic separation (IMS) and immunofluorescent antibody staining, with 4',6-diamidino-2-phenylindole (DAPI) staining for confirmation of oocysts. The entire pellet produced from each sample can be processed and examined. Packed pellets may range in size from <0.1 to 1 mL in volume, with those >0.5 mL being split into 2 IMS tests (as per the manufacturer's instructions).

EasySeed^{™} (containing 98 ± 1.4 *Cryptosporidium* oocysts and 98 ± 1.4 *Giardia* cysts) may be spiked into some samples immediately prior to IMS to quantify losses associated with the IMS and staining procedures. EasySeed^{™} vials may be vortex mixed for 30 s before being added to an IMS tube. One millilitre of the 10x SL-buffer A from the IMS kit may then added to the vial, vortex mixed for 30 s, and the washings added to the IMS tube. One millilitre of the 10x SL-buffer B from the IMS kit may then be added to the vial, vortex mixed for 30 s, and decanted into the IMS tube. The sample concentrate may then be added to the IMS tube.

IMS may be performed using the Dynabeads GC-Combo kit (Dynal, Oslo) according to the manufacturer's instructions, except that following the acid dissociation step, the sample may immediately be transferred to a 13-mm polycarbonate membrane filter of 0.8µm pore size (Nuclepore, Clifton, New Jersey) mounted on a vacuum manifold. The organisms on the membrane may be treated with methanol for 1 min and then overlaid for 2 min with 80 µL of DAPI (2 µg/mL in PBS) (Sigma-Aldrich). The DAPI may be removed by vacuum filtration and the membrane washed with 200 µL of EasyStain^{™} wash buffer (BTF Pty Ltd) and then overlaid for 15 min with 80 µL of EasyStain^{™} containing fluorescein isothiocyanate labelled antibodies specific for *Cryptosporidium* and *Giardia* (Weir et al. 2000 Clin. Diagn. Lab. Immunol. 7(5):745-750).

All staining may be performed at room temperature. The membrane may then be transferred to a slide, overlaid with the EasyStain^{™} mounting medium (BTF Pty Ltd), and sealed with a coverslip. The slide-mounted membrane may then be examined by epilfluorescence microscopy.

### Amplification of DNA or RNA within whole cells

A person skilled in the art will appreciate that this technique may be applied to the *in situ* detection of nucleic acids in whole cells as described, for example, in Prescott et al., 1999, Lett. Appl. Microbiol. 29: 396 - 400.

### EXAMPLES

### Example 1: Sample source and DNA purification

*Cryptosporidium* DNA was obtained from purified oocyst samples originating from culture or faeces. Oocysts from faeces were purified using a QIAamp DNA Stool kit (Qiagen, CA, USA) while oocysts from culture were subjected to a single PBS wash. Purified oocysts were suspended in 1x PCR buffer II (10 mM Tris-HCl, pH 8.3, 50 mM KCl) (Applied Biosystems, CA, USA), enumerated by haemacytometer cell chamber count and subjected to five cycles of freeze/thawing. The samples were then subjected to centrifugation at 10,000 xg for 5 min and the supernatant transferred to a fresh microcentrifuge tube. Serial dilutions (1:2) were then made of oocyst samples to desired concentrations, ranging from 10,000 to one equivalent oocyst/s per sample.

*Cryptosporidium* species or genotypes previously identified by other molecular techniques and used in this study were *C. hominis* isolates 6149, 6189, H270, H271, H273 (unpublished); *C. parvum* IOWA, AZ-1; *C. meleagridis* CZ-B1-32 (Ryan *et al.,* 2003); *C. andersoni* CZ-B1-52 (Ryan *et al.,* 2003); *C. baileyi* CZ-B1-15 (Ryan *et al.* 2003); *C. felis* 100.22 (unpublished); *C. galli* BE 13 (Ng *et al.,* 2006); *C. suis* WA Pig 6 (Ryan *et al.,* 2003); Marsupial genotype II (unpublished); and Mouse Genotype II (unpublished).

Negative controls utilized in this study were DNA purified from faecal samples and identified by other PCR methods as *Giardia duodenalis* (Assemblages A, B and E), *Isospora ohioensis, Sarcocystis* spp., *Ancylostuma caninum,* or *Spirometra erinaceieuropaei* (unpublished data).

### Example 2: LAMP Primers

The LAMP primers were designed for specificity to the *Cryptosporidium* actin gene. Previously published sequences (Sulaiman *et al.,* 2002) were aligned using *BioEdit* Version 7.0.5.2 (Hall, 1999), and a set of LAMP primers designed manually with the aid of the alignments and Eiken *Primer Explorer* Version 3 (http://primerexplorer.ip/e/). Primers were synthesized commercially by Sigma-Proligo (NSW, Australia).

**Table 1: The four LAMP primer nucleotide sequences targeting the C. parvum and C. hominis gene.**

| Primer | Sequence, 5'-3' | Nucleotide position | SEQ ID NO: |
|---|---|---|---|
| F3 | caagatgtgttttcccatcga | 83-103 | 1 |
| B3 | cctctggagcaacacgtaat | 282 - 301 | 2 |
| FIP | ctttgattgagcttcatcaccaacgccaggtgttatggtagg | 163 - 186 (F1c), | 7 |
| | | 123 - 140 (F2) | |
| BIP | cttgaaatacccaattgagcatgggttatagaaagtatgatgccagat c | 201-224(B1c), 255 - 278 (B2) | 8 |

Nucleotide position numbering is based upon the *C. hominis* TU502 actin sequence, GenBank XM_661095 (Xu *et al.,* 2004). Note that FIP and BIP, while each only constituting one primer, consist of two separate nucleotide regions, F1c and F2, and B1c and B2, respectively.

### Example 3: LAMP Reaction

The 25 µl LAMP reaction mixture consisted of 1.6 µM each of FIP and BIP inner primers, 0.8 µM each of F3 and B3 outer primers, 8U *Bst* enzyme (New England Biolabs, MA USA), 1× ThermoPol reaction buffer (New England Biolabs), 200 µM dNTP mix (Fisher-Biotech, Western Australia), 0.8 M Betaine (Sigma-Aldrich, CA, USA), 3.34 µpM SYTO9 (Molecular Probes, OR USA) and one µl of the heat-denatured DNA template (95°C for five minutes). The reaction mixture was loaded into a RotorGene 3000 (Corbett Research, NSW Australia) and run at 60°C for 60 min with fluorescent data acquisition for 30 seconds of every minute on the FAM channel (excitation at 470nm, detection at 510 nm). Three gain settings (1, 2 and 4 or 5) were included for the fluorescent data acquisition. The reaction was then terminated by incubating at 80°C for five minutes.

### Example 4: Amplicon detection and analysis

DNA amplified by the LAMP reaction was detected by two different methods, these being real-time fluorescent data acquisition and inspection under UV light of ethidium bromide stained 1.5% agarose gels after electrophoresis.

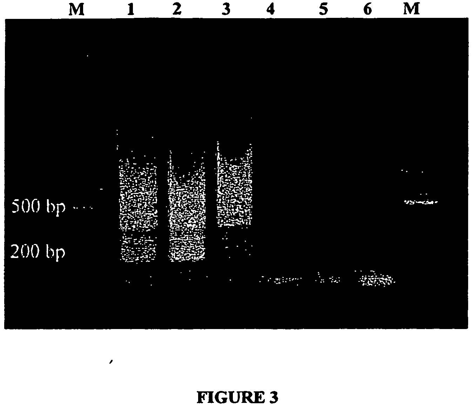

Amplicons were also sequenced in order to confirm that the *Cryptosporidium* LAMP primers had amplified the intended target. Briefly 10 µl of LAMP post-reaction DNA obtained from C. *hominis* 6149 template was subjected to gel electrophoresis. An approximately 180 bp fragment (Fig. 3) that was excised and purified was then subjected to PCR using the primers
5'-aggtgttatggtaggtatg-3' FseqCrAct (SEQ ID NO: 5)
   and
5'-agaaagtatgatgccagatc-3' BseqCrAct (SEQ ID NO: 6)
which contain sequences identical to regions overlapping the F2 and B2 sequences of the LAMP amplicon. The amplified product was cloned into a TOPO-TA vector (Invitrogen, CA, USA), transformed into *Escherichia coli,* and sequences obtained from clones after screening.

### Example 5: Detection of LAMP positives

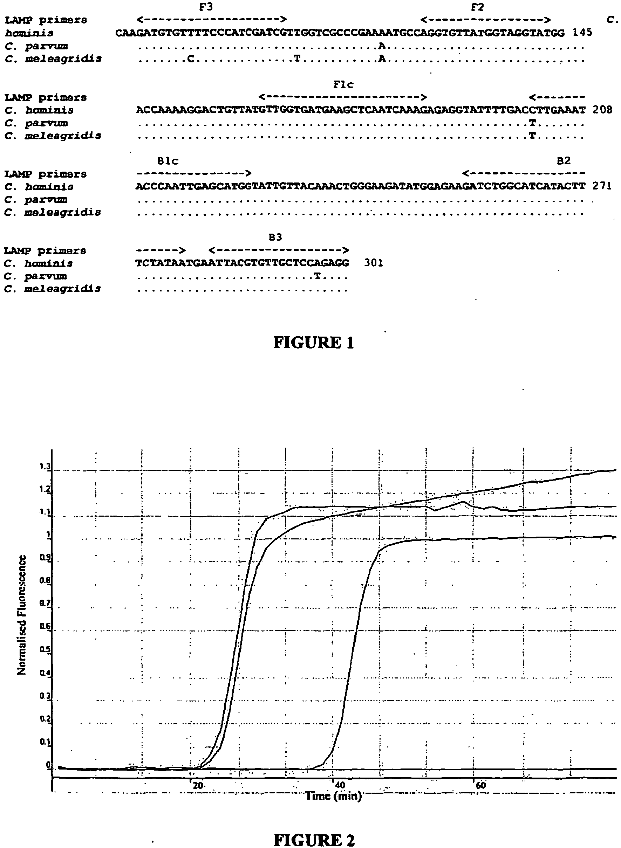

The LAMP primers were designed with specificity for *C. hominis,* with a one base-pair mismatch at the 5' end of the BIP primer. However, in practice the primers amplified the targeted actin gene of *C. hominis, C. parvum* as well as *C. meleagridis* while failing to amplify any other DNA that was applied. Detection of *C. hominis* and *C. parvum* by the described methods was made in approximately 30 to 35 minutes, while detection of *C. meleagridis* occurred in approximately 45 minutes (Fig. 2). The LAMP reaction failed to amplify DNA extracted from all other species tested, including other *Cryptosporidium* species. Sequencing of the cloned LAMP product produced an identical sequence to *C. hominis* 6149.

Positive LAMP reactions were also detected in ethidium bromide stained agarose gels in agreement with real-time results. Positive LAMP reactions produced a characteristic ladder-like appearance (Fig. 3), with the smallest band being approximately 180 bp in size.

It will be appreciated by persons skilled in the art that numerous variations and/or modifications may be made to the invention as shown in the specific embodiments.

## Claims

1. A method of detecting the presence of any one or more of *C. hominis, C. parvum* and *C. meleagridis* in a test sample, the method comprising contacting the test sample with:
(i) a first and a second outer primer, wherein the first outer primer is complementary to a region adjacent to the 5' terminus of a target DNA sequence and the second outer primer is complementary to a region adjacent to the 3' terminus of the target DNA sequence, and
(ii) a first and second inner primer, wherein the inner primers each contain two distinct sequences corresponding to a sense and antisense sequence of the target DNA sequence; and
performing auto-cycling strand displacement DNA synthesis using a loop-mediated isothermal amplification ("*LAMP*") method, and detecting the presence of amplified target DNA, wherein the target DNA sequence is a conserved DNA sequence encoding actin or parts thereof of *C. hominis, C. parvum* and *C. meleagridis.*

2. The method according to claim 1, wherein the detection is performed using a dsDNA intercalating fluorescent dye, including SYTO-9.

3. The method according to claim 1 or claim 2, wherein the auto-cycling is performed in the presence of a high strand displacement activity DNA polymerase.

4. Use of an amplification primer for detecting the presence of any one or more of *C. hominis, C. parvum* and *C. meleagridis* in an amplification assay, the amplification primer comprising an at least 10 contiguous base region which is at least 80% identical to an at least 10 contiguous base region present in any one of SEQ ID NOs 1 - 4
| | |
|---|---|
| caagatgtgttttcccatcga | SEQ ID NO: 1 |
| cctctggagcaacacgtaat | SEQ ID NO: 2 |
| ctttgattgagcttcatcaccaacngccaggtgttatggtagg | SEQ ID NO: 3 |
| cttgaaatacccaattgagcatggnttatagaaagtatgatgccagatc | SEQ ID NO: 4, |
wherein n is tttt or not present.

## Patentansprüche

1. Verfahren zum Nachweis der Anwesenheit eines oder mehrerer der Parasiten C. hominis, C. parvum und C. meleagridis in einer Untersuchungsprobe, wobei das Verfahren aufweist: Inkontaktbringen der Untersuchungsprobe mit:
(i) einem ersten und einem zweiten äußeren Primer, wobei der erste äußere Primer komplementär zu einer an den 5'-Terminus einer Target-DNA-Sequenz angrenzenden Region ist und der zweite äußere Primer komplementär zu einer an den 3'-Terminus der Target-DNA-Sequenz angrenzenden Region ist, und
(ii) einem ersten und einem zweiten inneren Primer, wobei die inneren Primer jeweils zwei getrennte Sequenzen enthalten, die einer Sense- und einer Antisense-Sequenz der Target-DNA-Sequenz entsprechen; und
Durchführen einer selbstauslösenden DNA-Synthese mit Strangverdrängung unter Anwendung eines loop-vermittelten isothermen Amplifikationsverfahrens (LAMP-Verfahren) und Nachweis der Anwesenheit von amplifizierter Target-DNA, wobei die Target-DNA-Sequenz eine konservierte DNA-Sequenz ist, die für Actin oder Teile davon von C. hominis, C. parvum und C. meleagridis codiert.

2. Verfahren nach Anspruch 1, wobei der Nachweis mit einem interkalierenden dsDNA-Fluoreszenzfarbstoff geführt wird, einschließlich SYTO-9.

3. Verfahren nach Anspruch 1 oder Anspruch 2, wobei die Selbstauslösung in Anwesenheit einer DNA-Polymerase mit hoher Strangverdrängungsaktivität erfolgt.

4. Verwendung eines Amplifikationsprimers zum Nachweis der Anwesenheit eines oder mehrerer der Parasiten C. hominis, C. parvum und C. meleagridis in einem Amplifikationsassay, wobei der Amplifikationsprimer eine Region von mindestens 10 zusammenhängenden Basen aufweist, die mit einer in einer der SEQ ID Nm.: 1-4 anwesenden Region von mindestens 10 zusammenhängenden Basen zu mindestens 80% identisch ist:
| | |
|---|---|
| caagatgtgttttcccatcga | SEQ ID Nr.: 1 |
| cctctggagcaacacgtaat | SEQ ID Nr.: 2 |
| ctttgattgagcttcatcaccaacngccaggtgttatggtagg | SEQ ID Nr.: 3 |
| cttgaaatacccaattgagcatggnttatagaaagtatgatgccagatcSEQ | ID Nr.: 4, |
wobei n gleich tttt oder nicht vorhanden ist.

## Revendications

1. Procédé pour la détection de la présence de l'un quelconque ou plus parmi C. *hominis, C. parvum* et *C. meleagridis* dans un échantillon d'essai, le procédé comprenant la mise en contact de l'échantillon d'essai avec:
(i) des première et deuxième amorces externes, où la première amorce externe est complémentaire à une région adjacente à la terminaison 5' d'une séquence d'ADN cible et la deuxième amorce externe est complémentaire à une région adjacente à la terminaison 3' de la séquence d'ADN cible, et
(ii) des première et deuxième amorces internes, où les amorces internes contiennent chacun deux séquences distinctes correspondant à une séquence sens et une séquence anti-sens de la séquence d'ADN cible; et
la réalisation d'un cycle automatique de synthèse d'ADN par déplacement de brins en utilisant une méthode d'amplification isothermique induite par boucle (« LAMP »), et la détection de la présence d'ADN cible amplifié, où la séquence d'ADN cible est une séquence d'ADN conservée codant pour l'actine ou des parties de celle-ci de *C. hominis, C. parvum* et *C. meleagridis.*

2. Procédé selon la revendication 1, dans lequel la détection est effectuée en utilisant un colorant fluorescent d'intercalation d'ADNds (double brin), incluant SYTO-9.

3. Procédé selon la revendication 1 ou la revendication 2, dans lequel le cycle automatique est effectué en présence d'une ADN-polymérase à activité de déplacement de brins élevée.

4. Utilisation d'une amorce d'amplification pour la détection de la présence de l'un quelconque ou plus parmi *C. hominis, C. parvum* et *C. meleagridis* dans un dosage d'amplification, l'amorce d'amplification comprenant une région d'au moins 10 bases contiguës qui est au moins 80% identique à une région d'au moins 10 bases contiguës présente dans l'une quelconque des SEQ ID NOs 1-4:
| | |
|---|---|
| caagatgtgttttcccatcga | SEQ ID NO:1 |
| cctctggagcaacacgtaat | SEQ ID NO:2 |
| ctttgattgagcttcatcaccaacngccaggtgttatggtagg | SEQ ID NO:3 |
| cttgaaatacccaattgagcatggnttatagaaagtatgatgccagatc | SEQ ID NO:4, |
dans lesquelles n est tttt ou n'est pas présent.
